# EUROPEAN PATENT APPLICATION

(11) **EP 4 656 235 A1**
(43) Date of publication of application: **03.12.2025**
(21) Application number: 24883846.8
(22) Date of filing: 09.05.2024
(51) Int. Cl.: A61N 1/40, A61N 1/02, A61N 1/08

(54) **TREATMENT HEAD, HANDPIECE, AND MICRONEEDLE TREATMENT DEVICE**

(30) Priority: 02.11.2023 CN 202322978391 U
(71) Applicant: Shenzhen Peninsula Medical Group, Shenzhen, Guangdong 518000 (CN)
(72) Inventor: LIANG, Yongsheng, Shenzhen, Guangdong 518000 (CN); ZHANG, Yuanchang, Shenzhen, Guangdong 518000 (CN); LI, Weihuo, Shenzhen, Guangdong 518000 (CN); LEI, Xiaobing, Shenzhen, Guangdong 518000 (CN)
(74) Representative: Osterhoff, Utz
(86) International application number: PCT/CN2024/092035
(87) International publication number: WO 2025/091833

(57) **Abstract**

Disclosed are a treatment tip, a hand tool and a needle treatment device. A treatment tip includes a needle plate portion, an adapter board portion and a first flexible circuit board. The needle plate portion is provided with a plurality of needles. The needle plate portion is slidably connected to the adapter board portion. The driving end of the handpiece drives the needle plate portion to move in the first direction relative to the adapter board portion. The first flexible circuit board is connected to the needle plate portion and the adapter board portion respectively. The radio frequency energy output by the handpiece passes through the adapter board portion, the first flexible circuit board and the needle plate portion, and the plurality of needles are controlled to generate heat in different zones. The present application is intended to increase the service life of the treatment tip, reduce the size of the treatment tip, and improve the stability and safety of the use of the treatment tip.

## Description

### CROSS-REFERENCE TO RELATED APPLICATIONS

The present application claims priority to Chinese Patent Application No. 202322978391.1, filed on November 2, 2023, the entire content of which is incorporated herein by reference.

### TECHNICAL FIELD

The present application relates to the technical field of medical devices, and in particular to a treatment tip, a hand tool and a needle treatment device.

### BACKGROUND

Radio frequency (RF) needle technology uses tiny needles to inject high-energy RF energy into target groups at different depths, thereby promoting the reorganization and regeneration of collagen. It can be used for facial rejuvenation applications such as skin tightening and scar removal. It can also be used for acne treatment, axillary hyperhidrosis treatment.

In the related art, there are many contacts on the treatment tip adapter plate. After the treatment tip is installed on the handpiece, the probe on the handpiece is connected to the pins on the adapter plate. Several needles are installed on the treatment tip, and each of the needles is connected to one pin on the adapter plate. At this time, the handpiece end can radiate different sequences of radio frequency energy to cause the needle treatment tip to apply the RF energy into different zones of the human body.

However, on the one hand, each of the needles needs to be coupled to the pin on the adapter plate through a separate wire, which easily causes the wire to break during the high-speed reciprocating movement of the needle plate. After the wire is broken, the needle cannot apply the RF energy in different zones, thus affecting the effect of RF treatment. On the other hand, providing wires that match the number of the needles will make the entire treatment tip larger, the spacing area among the needles, and the needle-density of the treatment tip cannot be guaranteed, resulting in poor aesthetics and less effectiveness. Secondly, the internal circuits are complex, and stability and safety cannot be effectively guaranteed.

### SUMMARY

The main purpose of the present application is to provide a treatment tip, which aims to increase the service life of the treatment tip, reduce the volume of the treatment tip, and improve the stability and safety of the usage of the treatment tip.

In order to achieve the above purpose, the treatment tip provided by the present application includes a needle plate portion, an adapter board portion and a first flexible circuit board; the needle plate portion has a plurality of needles; the needle plate portion is slidingly connected to the adapter board portion; a driving end of a handpiece is configured to drive the needle plate portion to move in a first direction relative to the adapter board portion; the first flexible circuit board is connected to the needle plate portion and the adapter board portion respectively; after radio frequency energy output by the handpiece sequentially is configured to pass through the adapter board portion, the first flexible circuit board and the needle plate portion, the plurality of needles are controlled to generate heat in different zones.

In an embodiment of the present application, a fixing assembly is configured to fix the first flexible circuit board to the needle plate portion and/or the adapter board portion.

In an embodiment of the present application, the needle plate portion includes a needle plate body and a guide member, and the plurality of the needles are installed on the needle plate body; the needle plate body is coupled with the first flexible circuit board, and a plurality of guide holes are provided on the adapter board portion; one end of the guide member is connected to the needle plate body, and the other end of the guide member passing through the guide holes is slidably connected to the adapter board portion.

In an embodiment of the present application, the fixing assembly includes a first fixing plate and a first fastener, and a first end of the first flexible circuit board is located between the first fixing plate and the guide member; a first opening is provided on the first fixing plate, and the first fastener is connected to the guide member by passing through the first opening.

In an embodiment of the present application, the adapter board portion includes an adapter board body and a connecting member connected with each other; the adapter board body is connected to the first flexible circuit board, and the fixing assembly is connected to the connecting member.

In an embodiment of the present application, the fixing assembly further includes a second fixing plate and a second fastener, and a second end of the first flexible circuit board is disposed between the second fixing plate and the connecting member; a second opening is provided on the second fixing plate, and the second fastener is connected to the connecting member by passing through the second opening.

In an embodiment of the present application, the needle plate body, the first flexible circuit board and the adapter board body are integrally formed.

In an embodiment of the present application, a length of the first flexible circuit board is greater than a distance between the needle plate body and the adapter board body.

In an embodiment of the present application, the treatment tip further includes a pressure block and an elastic member; the elastic member is sleeved on the guide member and is abutted against one end of the connecting member, and the pressure block is installed on the guide member and is abutted against the opposite end of the elastic member.

In an embodiment of the present application, the treatment tip further includes a housing, the housing includes a receiving cavity with an opening, and a plurality of needle holes are disposed at a bottom of the receiving cavity; the needle plate portion, the adapter board portion and the first flexible circuit board are disposed in the receiving cavity; the adapter board portion is fixed to the housing; and each needle is driven to moves inwards or outwards of the needle hole.

In an embodiment of the present application, the treatment tip further includes a negative pressure tube and a suction assembly; the negative pressure tube is provided on an outer peripheral wall of the housing and is communicated with the receiving cavity, and one end of the suction assembly is connected to the negative pressure tube.

In an embodiment of the present application, the housing includes transparent materials.

The present application also provides a hand tool, including a handpiece and the treatment tip as described above; the handpiece is detachably connected to the treatment tip; the handpiece includes a second flexible printed circuit, and the second flexible printed circuit is electrically connected to the treatment tip to allow the handpiece to radiate radio frequency energy to the treatment tip.

In an embodiment of the present application, the second flexible printed circuit includes an adapter unit, a first control unit and a second control unit, and the adapter unit, the first control unit and the second control unit are integrally formed.

The present application also provides a needle treatment device, including the hand tool as described above.

The technical solution of the present application includes a needle plate portion, an adapter board portion and a first flexible circuit board. The needle plate portion is provided with a plurality of needles. The needle plate portion is slidably connected to the adapter board portion. The needle plate portion moves forth and back in relation to the adapter board portion to realize the reciprocating movement of the needles in the treatment tip, thereby achieving the planar treatment effect of the treatment tip. The first flexible circuit board is electrically connected to the needle plate portion and the adapter board portion respectively. The radio frequency energy output by the handpiece passes through the adapter board portion, the first flexible circuit board and the needle plate portion, and the plurality of needles is controlled to generate heat in different zones. By connecting the needle plate portion and the adapter board portion through the flexible printed circuit, the radio frequency energy can be transmitted to several needles on the needle plate portion through the adapter board portion and the first flexible circuit board. Through connection of the flexible printed circuit, the integration effect of multiple lines can be achieved, high power transmission can be achieved, and the effect of controlling the heating of several needles in zones can be achieved. At the same time, compared with wire connections, the flexible printed circuit can withstand multiple repeated movements, thereby increasing the stability and safety of the treatment tip.

### BRIEF DESCRIPTION OF THE DRAWINGS

In order to more clearly explain technical solutions in the embodiments of the present application or in the related art, accompanying drawings required in the description of the embodiments or the related art will be briefly introduced below. Obviously, the accompanying drawings in the following description are only some embodiments of the present application. For those skilled in the art, other drawings can be obtained based on the structures shown in these drawings without creative efforts.
FIG. 1 is a schematic structural view of a treatment tip according to an embodiment of the present application.

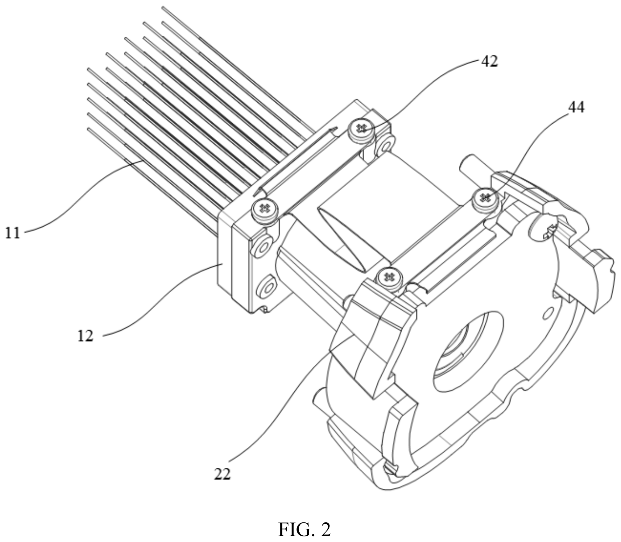

FIG. 2 is a schematic structural view of the treatment tip according to another embodiment of the present application.

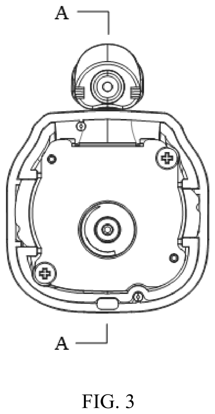

FIG. 3 is a top view of the treatment head according to another embodiment of the present application.

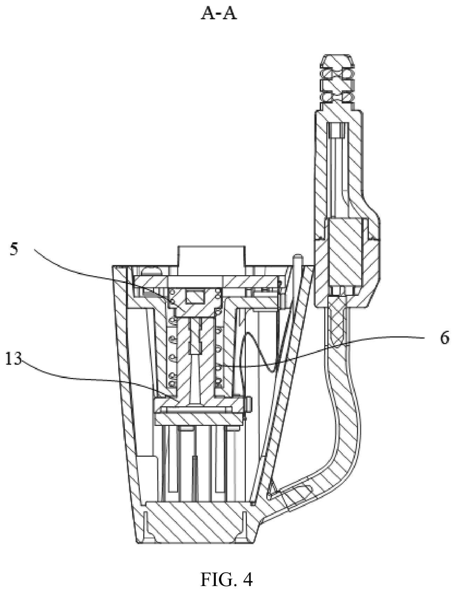

FIG. 4 is a schematic cross-sectional view of A-A in FIG. 3.

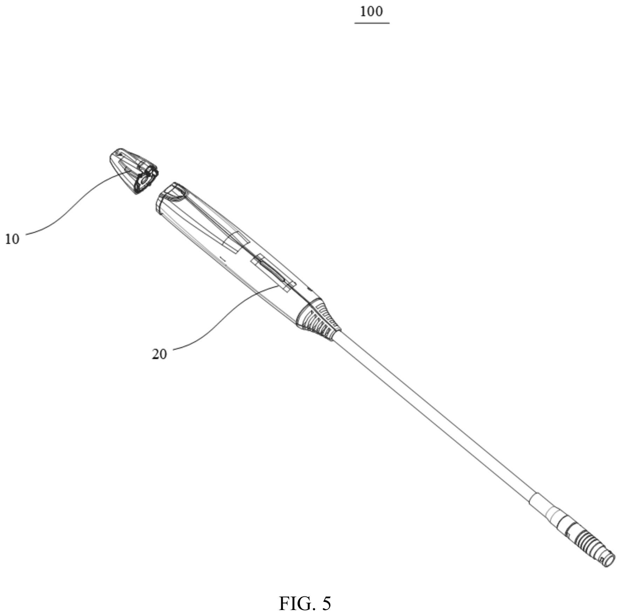

FIG. 5 is a schematic structural view of a hand tool according to an embodiment of the present application.

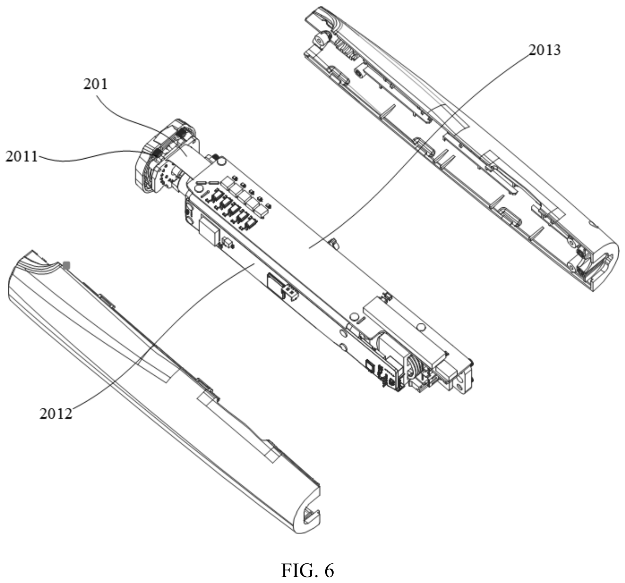

FIG. 6 is a schematic structural view of a handpiece of the hand tool according to an embodiment of the present application.

Description of reference signs:

**[Table 1]**

| reference sign | name | reference sign | name |
|---|---|---|---|
| 100 | hand tool | 43 | second fixing plate |
| 10 | treatment tip | 431 | second opening |
| 1 | needle plate portion | 44 | second fastener |
| 11 | needle | 5 | pressure block |
| 12 | needle plate body | 6 | elastic member |
| 13 | guide member | 7 | housing |
| 2 | adapter board portion | 71 | needle hole |
| 21 | adapter board body | 8 | negative pressure tube |
| 22 | connecting member | 9 | suction assembly |
| 3 | first flexible circuit board | 20 | handpiece |
| 4 | fixing assembly | 201 | second flexible printed circuit |
| 41 | first fixing plate | 2011 | adapter unit |
| 411 | first opening | 2012 | first control unit |
| 42 | first fastener | 2013 | second control unit |

The realization of the purpose, functional features and advantages of the present application will be further described in conjunction with the embodiments, with reference to the accompanying drawings.

### DETAILED DESCRIPTION OF THE EMBODIMENTS

The technical solutions in the embodiments of the present application will be clearly and completely described below with reference to the accompanying drawings in the embodiments of the present application. Obviously, the described embodiments are only some rather than all of the embodiments of the present application. Based on the embodiments in the present application, all other embodiments obtained by those skilled in the art without creative efforts fall within the scope of the present application.

It should be noted that all directional indications (such as up, down, left, right, front, back...) in the embodiments of the present application are only used to explain the relative positional relationship between components in a specific posture (as shown in the drawings), movement conditions, etc. If the specific posture changes, the directional indication will also change accordingly.

In addition, descriptions involving "first", "second", etc. in the present application are for descriptive purposes only and cannot be understood as indicating or implying their relative importance or implicitly indicating the number of indicated technical features. Therefore, features defined as "first" and "second" can explicitly or implicitly include at least one of these features. In addition, the technical solutions in the various embodiments can be combined with each other, but it must be based on what those skilled in the art can implement. When the combination of technical solutions is contradictory or cannot be realized, it should be considered that such a combination of technical solutions does not exist, nor is it within the scope of the present application.

The present application provides a treatment tip 10, aiming to increase the service life of the treatment tip 10, reduce the volume of the treatment tip 10, and improve the stability and safety of the use of the treatment tip 10.

In the embodiment of the present application, the treatment tip 10 includes a needle plate portion 1, an adapter board portion 2 and a first flexible circuit board 3. The needle plate portion 1 is provided with a plurality of needles 11. The needle plate portion 1 is slidably connected to the adapter board portion 2. A driving end in a handpiece 20 drives the needle plate portion 1 to move in a first direction relative to the adapter board portion 2. The first flexible circuit board 3 is connected to the needle plate portion 1 and the adapter board portion 2 respectively. The radio frequency energy output by the handpiece 20 passes through the adapter board portion 2, the first flexible circuit and the needle plate portion 1 in sequence, and controls the plurality of needles 11to generate heat in different zones.

The technical solution of the present application includes a needle plate portion 1, an adapter board portion 2 and a first flexible circuit board 3. The needle plate portion 1 and the adapter board portion 2 adopt PCBA, which can transmit the radio frequency energy to the plurality of needles 11, thereby enabling the plurality of needles 11 to generate heat for exothermic treatment of the site to be treated. The first flexible circuit board 3 has certain bending and stretching capabilities, and is not easily broken during the reciprocating movement of the needle board, causing circuit connection failure. The needle plate portion 1 is provided with a plurality of needles 11. The needle plate portion 1 is slidably connected to the adapter board portion 2. The needle plate portion 1 moves back and forth relative to the adapter board portion 2 to realize the reciprocating movement of the needles 11 back and forth, and the first direction is the first direction in the figure, thereby achieving the planar treatment effect of the treatment tip 10. The first flexible circuit board 3 is connected to the needle plate portion 1 and the adapter board portion 2 respectively. The radio frequency energy output by the handpiece 20 passes through the adapter board portion 2, the first flexible circuit and the needle plate portion 1, and controls several needles 11 to generated heat in different zones. By connecting the needle plate portion 1 and the adapter board portion 2 through the flexible printed circuit, the radio frequency energy can be transmitted to several needles 11 on the needle plate portion 1 through the adapter board portion 2 and the first flexible circuit board 3. Through connection of the flexible printed circuit, the integration effect of multiple lines can be achieved, high power transmission can be achieved, and the effect of controlling the heating of several needles 11 in zones can be achieved. At the same time, compared with wire connections, the flexible printed circuit can withstand multiple repeated movements, thereby increasing the service life of the treatment tip 10.

In an embodiment of the present application, the treatment tip 10 further includes a fixing assembly 4, which is used to partially fix the first flexible circuit board 3 to the needle plate portion 1 and/or the adapter board portion 2.

In this embodiment, two ends of the first flexible circuit board 3 are further reinforced with the needle plate portion 1 and the adapter board portion 2 respectively through the fixing assembly 4, so that connection strength between the first flexible circuit board 3 and the needle plate portion 1 and/or the adapter board portion 2 can be increased, which can increase the service life of the treatment tip 10.

In an embodiment of the present application, the needle plate portion 1 includes a needle plate body 12 and a guide member 13. A plurality of needles 11 are installed on the needle plate body 12. The needle plate body 12 is connected to the first flexible circuit board 3. The adapter board portion 2 is provided with a plurality of guide holes. One end of the guide member 13 is connected to the needle plate body 12, and the other end of the guide member 13 passes through the guide holes and is slidably connected to the adapter board portion 2.

In this embodiment, the needle plate portion 1 includes a needle plate body 12 and a guide member 13. The adapter board portion 2 is provided with a guide hole. One end of the guide member 13 is provided on the other side of the needle plate body 12 away from several needles 11, and the other end passes through the guide hole and is slidably connected to the adapter board portion 2. The cooperative connection between the guide hole and the guide member 13 can limit the sliding trajectory of the needle plate body 12 to allow the needle plate body 12 to move forth and back without deflecting sideways, preventing the treatment effect of the treatment tip 10 from being affected due to the skewed needle plate body 12.

In an embodiment of the present application, the fixing assembly 4 includes a first fixing plate 41 and a first fastener 42. A first end of the first flexible circuit board 3 is located between the first fixing plate 41 and the guide member 13. The fixing plate 41 is provided with a first opening 411, and the first fastener 42 is connected to the guide member 13 by passing through the first opening 411.

In this embodiment, the fixing assembly 4 includes a first fixing plate 41 and a first fastener 42. One end of the first flexible circuit board 3 is provided between the first fixing plate 41 and the guide member 13. The first fixing plate 41 is provided with a first opening 411, and the first fastener 42 passes through the first opening 411 and is connected to the guide member 13, thereby fixing the first fixing plate 41 to the guide member 13 and fixing one end of the first flexible circuit board 3.

In an embodiment of the present application, the adapter board portion 2 includes an adapter board body 21 and a connecting member 22. The adapter board body 21 is connected to the connecting member 22. The adapter board body 21 is connected to the first flexible circuit board 3. The fixing assembly 4 is connected to the connecting member 22.

In this embodiment, the adapter board portion 2 is connected to the adapter board body 21 and the connecting member 22. The adapter board body 21 is connected to the connecting member 22. The other end of the first flexible circuit board 3 is connected to the adapter board body 21, and the fixing assembly 4 is connected to the connecting member 22 to improve the connection strength between the adapter board portion 2 and the other end of the first flexible circuit board 3, thereby increasing the service life of the treatment tip 10.

In an embodiment of the present application, the fixing assembly 4 also includes a second fixing plate 43 and a second fastener 44. A second end of the first flexible circuit board 3 is disposed between the second fixing plate 43 and the connecting member 22. The second fixing plate 43 is provided with a second opening 431, and the second fastener 44 is connected to the connecting member 22 by passing through the second opening 431.

In this embodiment, the first fastener 42 passes through the first opening 411 and is connected to the connecting member 22 to connect and fix the other end of the first flexible circuit board 3 with the adapter board portion 2 to further enhance the connection strength between the adapter board portion 2 and the other end of the first flexible circuit board 3, further improving the service life of the treatment tip 10.

In an embodiment of the present application, the needle plate body 12, the first flexible circuit board 3 and the adapter board body 21 are integrally formed.

In this embodiment, the needle plate body 12, the first flexible circuit board 3 and the adapter board body 21 are integrally formed, which can facilitate processing, improve the connection strength between the three, and further prevent the connection between the three from breaking when the needle plate moves. The connection between the first flexible circuit board 3, the needle plate body 12 and the adapter board body 21 adopts a three-layer structure of hard, soft and hard. The flexible printed circuit is integrally formed with two hard boards, and the hard board is TG150.

In an embodiment of the present application, the length of the first flexible circuit board 3 is greater than a distance between the needle plate body 12 and the adapter board body 21.

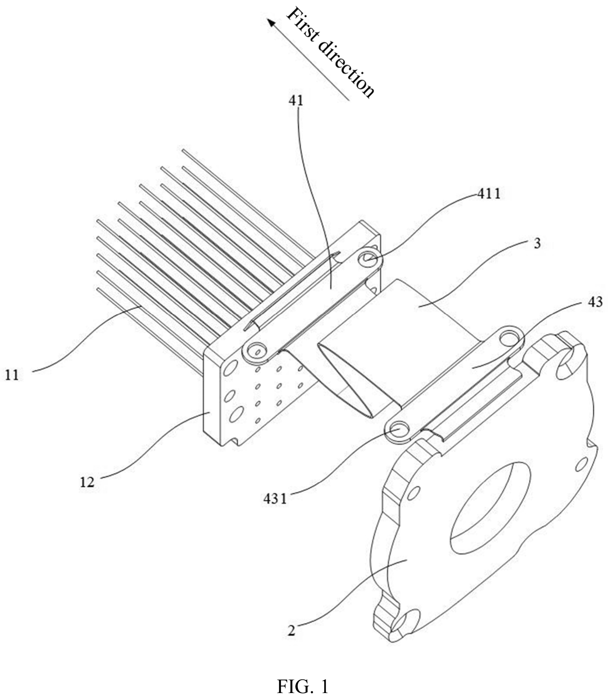

In this embodiment, the first flexible circuit board 3 is placed in the treatment tip 10 in an "S" shape. The "S" shape can effectively utilize the internal space of the treatment tip 10 and reduce the volume of the treatment tip 10. During the treatment process, the needle plate body 12 moves in the first direction as shown in FIG. 1, causing the first flexible circuit board 3 to extend. The length of the first flexible circuit board 3 is greater than the distance between the needle plate body 12 and the adapter board body 21, which can prevent the first flexible circuit board 3 from being pulled to damage the connection between the first flexible circuit board 3 and the needle plate body 12 or the adapter board body 21, thereby causing the treatment tip 10 to fail.

In an embodiment of the present application, the treatment tip 10 also includes a pressure block 5 and an elastic member 6. The elastic member 6 is sleeved on the guide member 13 and is abutted against one end of the connecting member 22. The pressure block 5 is installed on the guide member 13 and is abutted against the opposite end of the elastic member 6.

In this embodiment, the elastic member 6 is sleeved on the guide member 13 and is abutted against one end of the connecting member 22. The pressure block 5 is provided on the guide member 13. The pressure block 5 and the guide member 13 are connected through screws. A through hole is provided on the pressure block 5, and the pressure block 5 is fixed to the guide member 13 through screws and is abutted against the other end of the elastic member 6 to push the pressure block 5, so that the guide member 13 moves back and forth, thereby driving the needle plate body 12 to move back and forth, while squeezing the elastic member 6. After the treatment is completed, the pressure block 5 is no longer pushed, the elastic member 6 recovers its deformation, and drives the needle plate body 12 to return to its original position, realizing automatic reset of the needle plate body 12.

In an embodiment of the present application, the treatment tip 10 further includes a housing 7. The housing 7 includes a receiving cavity with an opening, and a plurality of needle holes are disposed at the bottom of the receiving cavity. The needle plate portion 1, the adapter board portion 2 and the first flexible circuit board 3 are embedded in the receiving cavity and are connected to the housing 7. Each needle 11 moves in or out of the needle hole.

In this embodiment, the housing 7 is made of medical-grade PC plastic, which is safe and non-toxic, and is transparent. The needle plate portion 1, the adapter board portion 2 and the needle 11 inside the housing 7 can be observed. The transverse cross-sectional shape of the housing 7 is rectangular, which matches the shape of the array formed by a plurality of needles 11. The cross-sectional area of the housing 7 gradually increases from the needle outlet end to the other end, which facilitates the movement of the needle plate portion 1 inside the receiving cavity of the housing 7 without being restricted by the housing 7, making the needle extraction of the treatment tip 10 smoother. Secondly, the housing 7 made of transparent plastic can facilitate the user to initially judge whether the treatment tip 10 is normal before use. For example, the user can observe whether the end of the first flexible circuit board 3 is broken or not, so as to ensure the safety of subsequent use.

In an embodiment of the present application, the treatment tip 10 is also provided with a negative pressure tube 8 and a suction assembly 9. The negative pressure tube 8 is provided on the outer peripheral wall of the housing 7 and communicates with the receiving cavity. One end of the suction assembly 9 is connected to the negative pressure tube 8.

In this embodiment, the treatment tip 10 is also provided with a negative pressure tube 8 that communicates with the receiving cavity. Through the negative pressure tube 8, the receiving cavity can be evacuated to a near vacuum state, so that the treatment tip 10 can be adsorbed on the skin surface of a patient, which can reduce the air resistance when the needle 11 breaks through the skin of the patient, and can absorb the skin of the treatment area, so that the depth of penetration of the needle 11 is consistent. The treatment tip 10 also includes a suction assembly 9, which includes a silicone tube, an upper filter shell, a lower filter shell, an absorbent sponge and a sealing ring. One end of the silicone tube is connected to the negative pressure tube 8, and the other end is connected to the upper filter shell. The upper filter shell and the lower filter shell are enclosed to form the receiving cavity. The absorbent sponge is provided in the receiving cavity to absorb the blood or sweat of the patient to prevent secondary pollution. The lower filter shell is connected to an air extraction device, and a sealing ring is provided at the connection to prevent air leakage.

The present application also provides a hand tool 100. The hand tool 100 includes a handpiece 20 and a treatment tip 10 as described above. The specific structure of the treatment tip 10 refers to the above-mentioned embodiments. Since the hand tool 100 adopts all the technical solutions of the above-mentioned embodiments, it has at least all the beneficial effects brought by the technical solutions of the above-mentioned embodiments, which will not be repeated here. The handpiece 20 is detachably connected to the treatment tip 10. The handpiece 20 includes a second flexible printed circuit 201. The second flexible printed circuit 201 is electrically connected to the treatment tip 10, so that the handpiece 20 radiates radio frequency energy to the treatment tip 10.

In this embodiment, a detachable connection is used between the handpiece 20 and the treatment tip 10 to realize rapid replacement of treatment tips of different specifications and improve treatment efficiency. The handpiece 20 and the treatment tip 10 are connected through the second flexible printed circuit to transmit radio frequency energy from the handpiece 20 to the treatment tip 10. By providing the second flexible printed circuit 201, the number of needles 11 provided on the handpiece 20 can be reduced accordingly. The number of contacts on the handpiece 20 is also reduced accordingly, and the number of needles 11 matches the number of contacts. Different groups can be divided according to the different heating forms of the needle 11, and each group is connected to a passage. It is only necessary to provide a corresponding number of needles 11 according to the number of groups required for zone heating to achieve zone heating. The energy transmitted by the second flexible printed circuit 201 of the handpiece 20 can enter the adapter board portion 2 through different passages, and after passing through the first flexible circuit board 3 and the needle plate portion 1, the needle plate portion 1 transmits the energy to different needles 11 and generates heat in different zones through different needles 11.

In an embodiment of the present application, the second flexible printed circuit 201 includes an adapter unit 2011, a first control unit 2012 and a second control unit 2013. The adapter unit 2011, the first control unit 2012 and the second control unit 2013 are integrally formed.

In this embodiment, the integrated molding of the adapter unit 2011, the first control unit 2012 and the second control unit 2013 can facilitate processing, and at the same time improve the connection strength between the three, further prevent the connection between the three from breaking, and thereby increasing the service life of the treatment device. Secondly, the adapter unit 2011, the first control unit 2012 and the second control unit 2013 are made of flexible material. The first control unit 2012 and the second control unit 2013 can be distributed on two adjacent sides of the handpiece 20, and the adapter unit 2011 can be located on an end surface of the handpiece 20 close to the treatment tip 10. Therefore, the flexible adapter unit 2011, the first control unit 2012 and the second control unit 2013 can be well stored in the internal space of the handpiece 20, thereby avoiding difficulty in operation caused by the handpiece 20 being made too large.

The present application also provides a needle treatment device, including the hand tool 100 as described above.

Since the needle treatment device according to the embodiment of the present application has the hand tool 100 in any of the above embodiments, it has the beneficial effects brought by the hand tool 100 in any of the above embodiments, which will not be described again here.

The embodiments of the present application have been described above, but those skilled in the art should know that the present application is not limited to the above-mentioned embodiments. The above-mentioned embodiments are only examples. Within the scope of the present application, embodiments that have substantially the same configuration as the technical idea and exert the same functions and effects are included in the scope of the present application. In addition, within the scope that does not deviate from the gist of the present application, various modifications that can be thought of by those skilled in the art are added to the embodiments, and other embodiments constructed by combining some of the constituent elements in the embodiments are also included in the scope of the present application.

## Claims

1. A treatment tip, **characterized by** comprising:
a needle plate portion, having a plurality of needles;
an adapter board portion, wherein the needle plate portion is slidably connected to the adapter board portion, and a driving end of a handpiece is configured to drive the needle plate portion to move in a first direction relative to the adapter board portion; and
a first flexible circuit board connected to the needle plate portion and the adapter board portion respectively, wherein after radio frequency energy output by the handpiece sequentially through the adapter board portion, the first flexible circuit board and the needle plate portion, and the plurality of needles are controlled to generate heat in different zones.

2. The treatment tip according to claim 1, further comprising:
a fixing assembly configured to fix the first flexible circuit board to the needle plate portion and/or the adapter board portion.

3. The treatment tip according to claim 2, wherein the needle plate portion comprises a needle plate body and a guide member, and the plurality of the needles are installed on the needle plate body; the needle plate body is coupled with the first flexible circuit board, and a plurality of guide holes are provided on the adapter board portion; one end of the guide member is connected to the needle plate body, and the other end of the guide member passing through the guide holes is slidably connected to the adapter board portion.

4. The treatment tip according to claim 3, wherein the fixing assembly comprises a first fixing plate and a first fastener, and a first end of the first flexible circuit board is disposed between the first fixing plate and the guide member; a first opening is provided on the first fixing plate, and the first fastener is connected to the guide member by passing through the first opening.

5. The treatment tip according to claim 3, wherein the adapter board portion comprises an adapter board body and a connecting member connected with each other; the adapter board body is connected to the first flexible circuit board, and the fixing assembly is connected to the connecting member.

6. The treatment tip according to claim 5, wherein the fixing assembly further comprises a second fixing plate and a second fastener, and a second end of the first flexible circuit board is disposed between the second fixing plate and the connecting member; a second opening is provided on the second fixing plate, and the second fastener is connected to the connecting member by passing through the second opening.

7. The treatment tip according to claim 5, wherein the needle plate body, the first flexible circuit board and the adapter board body are integrally formed.

8. The treatment tip according to claim 5, wherein a length of the first flexible circuit board is greater than a distance between the needle plate body and the adapter board body.

9. The treatment tip according to claim 5, further comprising:
a pressure block; and
an elastic member;
wherein the elastic member is sleeved on the guide member and is abutted against one end of the connecting member, and the pressure block is installed on the guide member and is abutted against the opposite end of the elastic member.

10. The treatment tip according to claim 1, further comprising: a housing, the housing comprising a receiving cavity with an opening; a plurality of needle holes disposed at a bottom of the receiving cavity; the needle plate portion, the adapter board portion and the first flexible circuit board are disposed in the receiving cavity and connected to the housing, and each needle is driven to move inwards or outwards of the needle hole.

11. The treatment tip according to claim 10, further comprising:
a negative pressure tube; and
a suction assembly;
wherein the negative pressure tube is provided on an outer peripheral wall of the housing and is communicated with the receiving cavity, and one end of the suction assembly is connected to the negative pressure tube.

12. The treatment tip according to claim 10, wherein the housing comprises transparent materials.

13. A hand tool, **characterized by** comprising:
a handpiece; and
the treatment tip according to any one of claims 1 to 12;
wherein the treatment tip is detachably connected to the handpiece; the handpiece comprises a second flexible circuit board, and the second flexible printed circuit is electrically connected to the treatment tip to allow the handpiece to radiate RF energy to the treatment tip.

14. The hand tool according to claim 13, wherein the second flexible printed circuit comprises an adapter unit, a first control unit and a second control unit, and the adapter unit, the first control unit and the second control unit are integrally formed.

15. A needle treatment device, **characterized by** comprising the hand tool according to claim 13 or 14.
